# EUROPÄISCHE PATENTSCHRIFT

(11) **EP 3 170 519 B1**
(45) Veröffentlichungstag und Bekanntmachung des Hinweises auf die Patenterteilung: **09.03.2022**
(21) Anmeldenummer: 16197526.3
(22) Anmeldetag: 07.11.2016
(51) Int. Cl.: A61M 1/16, A61L 2/18, B01D 65/02, B01D 65/10

(54) **VERFAHREN ZUR STERILISATION UND INTEGRITÄTSPRÜFUNG VON DIALYSATOREN**
METHOD FOR STERILIZING AND INTEGRITY VERIFICATION OF DIALYZERS
PROCÉDÉ DE STÉRILISATION ET DE CONTRÔLE D'INTÉGRITÉ DE DIALYSEURS

(30) Priorität: 18.11.2015 DE 102015120003
(43) Veröffentlichungstag der Anmeldung: 24.05.2017
(73) Patentinhaber: B. Braun Avitum AG, 34212 Melsungen (DE)
(72) Erfinder: Dr. BURKERT, Sina, 01157 Dresden (DE)
(74) Vertreter: Winter, Brandl - Partnerschaft mbB

(56) Entgegenhaltungen:
- EP-A2- 0 035 405
- DE-B4- 19 655 227
- US-A- 4 695 385
- US-A- 5 252 213
- US-A- 5 808 181
- William A Rutala ET AL: "Guideline for Disinfection and Sterilization in Healthcare Facilities Guideline for Disinfection and Sterilization in Healthcare Facilities, 2008 , and the Healthcare Infection Control Practices Advisory Committee (HICPAC)", , 1. Januar 2008 (2008-01-01), XP055166062, Gefunden im Internet: URL:http://www.cdc.gov/hicpac/pdf/guidelin es/Disinfection_Nov_2008.pdf [gefunden am 2015-01-30]

## Beschreibung

Die vorliegende Erfindung betrifft ein Verfahren zur Sterilisation und Integritätsprüfung von Dialysatoren.

Funktionsprüfung und Sterilisation medizinischer Geräte sind wesentliche Voraussetzungen, um eine optimale Versorgung von Patienten zu gewährleisten und eine Übertragung infektiöser Pathogene auf den Patienten zu verhindern.

Bei den heute in der modernen Medizin zur Hämodialyse und Hämo(dia)filtration verwendeten Dialysatoren, insbesondere Hohlfaserdialysatoren, bei denen die vom Blut durchströmten Hohlfasern die Filtermembran bilden, erfolgen Integritätsprüfung und Sterilisation in mehreren, voneinander unabhängigen Schritten.

Bei einem Hohlfaserdialysator wird im Verlauf des Produktionsprozesses nach den Prozessschritten Einzug des Faserbündels in das Dialysatorgehäuse, Faserversiegelung und Montage der Blutkappen in einem ersten Prüfschritt zunächst die Dichtigkeit der Fasern geprüft.

Hierzu wird der Dialysator in einer Nassdichteprüfung gewöhnlich zunächst mit destilliertem oder deionisiertem Wasser, insbesondere mit durch Umkehrosmose deionisiertem Wasser (RO-Wasser), gespült und einer Integritätsprüfung, beispielsweise einem Bubble-Point-Test oder Diffusionstest, unterzogen, um die Dichtigkeit der Hohlfasern festzustellen. Eine Integritätsprüfung ist grundlegender Bestandteil der EN ISO 8637:2014, Abschnitt 4.4.2 Unversehrtheit des Blutbereichs. Verfahren zum Testen der Integrität von Dialysatormembranen sind beispielsweise in US 5 594 161 A1 und WO 2012/020048 A1 beschrieben.

Sofern der Dialysator den Test besteht, wird das Wasser durch Erhitzen entfernt, beispielsweise durch eine Mikrowellentrocknung mit anschließender Trocknung durch vorab über Sterilfilter gereinigte Heißluft.

Da die Trocknung bei Temperaturen von maximal etwa 100°C erfolgt, die zur Sterilisation des Dialysators nicht ausreichen, wird der Dialysator anschließend einem separaten Sterilisationsprozess unterzogen, um pathogene Keime und Sporen abzutöten.

WO 2014/128306 beschreibt eine Prüf- und Trocknungseinrichtung zur Funktionsprüfung von Dialysatoren. Zur Nassdichteprüfung mit diesen Einrichtungen kann der Dialysator durch Anschlüsse für Luft und flüssiges Medium dialysierflüssigkeitsseitig mit Wasser und blutseitig mit Trockenluft durchströmt werden, wobei die Dichtigkeit der Fasern getestet wird. Durch diese Anschlüsse erfolgt anschließend eine Lufttrocknung des Dialysators. Nach Funktionsprüfung und weiterer Trocknung muss der Dialysator einem anschließenden Sterilisationsprozess unterworfen werden.

Weiter offenbart die Druckschrift US 4 695 385 ein wiederverwendbares Dialysesystem zum Reinigen, Sterilisieren und Testen einer Hämodialysemaschine und ihres zugehörigen Dialysators und Blutschlauchsets. Zur Sterilisation wird dabei Formaldehyd eingesetzt und ein Membrantest mittels einer Leitfähigkeitsmessung durchgeführt.

Die Druckschrift EP 0 035 405 A2 offenbart eine Vorrichtung und ein Verfahren zum Reinigen von Dialysatoren. Dabei werden die Blutseite und die Dialysierflüssigkeitsseite des Dialysators mit verschiedenen Chemikalien behandelt und die Dialysatormembran unter Verwendung von Druckluft getestet.

In der Druckschrift US 5 252 213 A ist ein automatisches Hämodialysesystem offenbart, in dem der Blutkreislauf einschließlich Dialysator vor dem Wiedergebraucht gereinigt, gespült, auf Integrität getestet und mit Sterilisationsmitteln befüllt wird.

Verfahren und Vorrichtungen zum Trocknen von Dialysatoren mittels Mikrowellentrocknung sind beispielsweise aus DE 10 2006 051 656 A1 bekannt. Auch hier muss der Dialysator nach der Trocknung noch einem anschließenden Sterilisationsprozess ausgesetzt werden.

Die Sterilisation von Dialysatoren erfolgt nach dem Stand der Technik entweder chemisch durch Sterilisation mit Ethylenoxid (EtO) oder Formalinlösung oder durch Strahlensterilisation, insbesondere Gammastrahlung, wobei die Bioburden-Bedingungen nach EN ISO 11137-2013 (D) erfüllt werden müssen. Die chemischen Sterilisationsverfahren haben jedoch den Nachteil, dass nach der Sterilisation toxische Rückstände im Dialysator verbleiben, die vor Anwendung des Dialysators erst durch gründliches Spülen entfernt werden müssen.

Eine Strahlensterilisation des Dialysators erfolgt in der Regel nicht durch den Hersteller selbst, sondern durch einen Fremddienstleister, und hat in jedem Fall den Nachteil eines hohen logistischen Aufwands. Weiterer Nachteil der Strahlensterilisation ist die durch die Strahlung induzierte Belastung der Materialien, z.B. Abbau der Materialien, sowie die Bildung möglicher toxischer Bestandteile. Für die Sterilisation von Blutschlauchsystemen ist auch die Sterilisation mit Wasserstoffperoxid-Plasma bekannt, die jedoch für die Sterilisation von Dialysatoren keine Anwendung findet.

Die bekannten Verfahren zur Integritätsprüfung von Dialysatoren in Verbindung mit chemischen Sterilisationsverfahren oder Strahlensterilisation sind zeitaufwendig und kostspielig.

Aufgabe der vorliegenden Erfindung ist es daher, ein einfaches und kostengünstiges Verfahren zur Sterilisation und Integritätsprüfung von Dialysatoren, beispielsweise Hohlfaserdialysatoren, bereitzustellen.

Diese Aufgabe wird mit dem Verfahren gemäß Anspruch 1 gelöst.

Folgende Schritte sind umfasst:
a) Behandeln des Dialysators mit einer Sterilisationsflüssigkeit zur Sterilisation wenigstens des blutseitigen Raums des Dialysators und zum Benetzen der Dialysemembran des Dialysators mit der Sterilisationsflüssigkeit; und
b) Durchführen einer Integritätsprüfung der mit der Sterilisationsflüssigkeit benetzten Dialysemembran,
wobei die Sterilisationsflüssigkeit ausgewählt ist aus:
(i) wässrigen Lösungen, die Peroxid und/oder Ozon enthalten, wobei das Peroxid ausgewählt ist aus Peroxiden, die sich zu Wasser, Sauerstoff und/oder flüchtigen organischen Verbindungen zersetzen; und/oder
(ii) Chlor, Brom und/oder Iod enthaltenden wässrigen Lösungen.

Weitere Ausführungsformen des erfindungsgemäßen Verfahrens sind in den Unteransprüchen angegeben.

Die erfindungsgemäß verwendeten Sterilisationsflüssigkeiten sind nicht nur geeignet, um den Dialysator zu sterilisieren, sondern können auch zur Vorbereitung und Durchführung der Funktionsprüfung eingesetzt werden. Auf diese Weise kann der Dialysator in Kombination mit der Sterilisation auf Integrität, insbesondere Dichtigkeit, der Dialysemembran, beispielsweise der Hohlfasern, geprüft werden.

Als Sterilisationsflüssigkeit können erfindungsgemäß wässrige Lösungen von Peroxiden und/oder Ozon und/oder wässrige Lösungen von Chlor, Brom und/oder Iod eingesetzt werden.

Als Peroxide eignen sich Peroxide, die sich bei Umgebungstemperatur oder bei erhöhten Temperaturen zu Wasser, Sauerstoff und/oder flüchtigen organischen Verbindungen zersetzen und so gasförmig und rückstandslos durch eine Dialysemembran wie eine Hohlfasermembran entweichen können. Unter flüchtigen organischen Verbindungen werden hierbei organische Verbindungen verstanden, die unter Normaldruck einen Siedepunkt von bis zu 120°C aufweisen.

Vorteilhaft enthält die als Sterilisationflüssigkeit eingesetzte peroxidhaltige wässrige Lösung Peroxide der Formel R¹-O-O-R², worin R¹ und R² unabhängig voneinander H, CH₃, C₂H₅ und CH₃C(O) bedeuten, und Mischungen davon. Geeignete Peroxide sind beispielsweise Wasserstoffperoxid (H₂O₂), Methylhydroperoxid (CH₃OOH), Ethylhydroperoxid (C₂H₅OOH), Dimethylperoxid ((CH₃)₂O₂), Dieethylperoxid ((C₂H₅)₂O₂), Ethylmethylperoxid (C₂H₅OOCH₃), Peroxyessigsäure (H₃CC(O)O-OH) und Mischungen davon. Besonders bevorzugt sind wässrige Wasserstoffperoxidlösungen, wobei das Wasserstoffperoxid in Lösung allein oder auch zusammen mit Peroxyessigsäure oder Ozon vorliegen kann. Im Hinblick auf arbeits- und umweltschutzrechtliche Aspekte bei der Produktion sind Wasserstoffperoxidlösungen die bevorzugte Wahl.

Als wässrige Peroxidlösungen können handelsübliche Peroxidlösungen verwendet werden. Geeignete Peroxidlösungen sind, ausgenommen Persäurelösungen, wenigstens 3%ige, beispielsweise 3 - 35%ige Peroxidlösungen. Vorteilhaft werden 5 - 30%ige Peroxidlösungen, bevorzugt 5 - 28%ige Peroxidlösungen, und besonders bevorzugt 5 - 25%ige Peroxidlösungen, insbesondere Wasserstoffperoxidlösungen, verwendet. Als Peroxyessigsäurelösungen werden zweckmäßig 0,1 - 0,5%ige Lösungen, bevorzugt 0,15 - 0,35%ige Lösungen verwendet. Wahlweise können Peroxidlösungen wie Wasserstoffperoxidlösungen verwendet werden, die geringe Mengen, beispielsweise 0,15 - 0,3%, Peroxyessigsäure enthalten. Der pH-Wert der wässrigen Peroxidlösungen liegt üblicherweise im sauren Bereich, vorteilhaft im Bereich von 2,0 bis 6,5, insbesondere im Bereich von 3,5 bis 5,0.

Geeignete wässrige Ozonlösungen haben beispielsweise einen Ozongehalt von 1 bis 3 ppm Ozon.

Chlor, Brom und/oder Iod enthaltende wässrige Lösungen haben üblicherweise einen Halogengehalt von 2% oder mehr. Wässrige Chlorlösungen sind bevorzugt.

Wässrige Peroxidlösungen, insbesondere wässrige Wasserstoffperoxidlösungen, mit oder ohne Peroxyessigsäure, sind besonders bevorzugt, da sich eine Behandlung mit diesen Lösungen positiv auf die Hämokompatibilität der Dialysatoroberflächen auswirkt. Die erfindungsgemäß verwendeten Sterilisationsflüssigkeiten sind dem Fachmann bekannt und im Handel erhältlich. Geeignete zugelassene Sterilisationsflüssigkeiten sind beispielsweise bei Rutala, W.A. et al., "Guideline for Disinfection and Sterilization in Healthcare Facilities, 2008" des Center of Disease Control (CDA) beschrieben. Behandlungen des Dialysators mit der wässrigen Sterilisationsflüssigkeit zur Sterilisation des Dialysators und zum Benetzen der Membran des Dialysators und Integritätsprüfung der mit der Sterilisationsflüssigkeit benetzten Membran können während des Produktionsprozesses und entsprechend den dem Fachmann bekannten Verfahrensschritten zur Integritätsprüfung mit Wasser erfolgen.

Im Einzelnen wird der Dialysator zunächst wenigstens auf der dem menschlichen Blut ausgesetzten Seite (Blutseite) des Dialysators mit der Sterilisationsflüssigkeit in Berührung gebracht. Dies kann durch Füllen und/oder Spülen des Dialysators mit der Sterilisationsflüssigkeit erfolgen. Erfindungsgemäß genügt es, die Blutseite des Dialysators mit der Sterilisationsflüssigkeit zu behandeln, da es nach heutigem regulatorischen Verständnis ausreicht, wenn nur der dem menschlichen Blut ausgesetzte Raum steril gehalten wird. Es werden sowohl Blutseite als auch Dialysierflüssigkeitsseite des Dialysators zur Sterilisation mit der Sterilisationsflüssigkeit behandelt.

Im Falle eines Hohlfaserdialysators wird die Sterilisationsflüssigkeit zur Sterilisation und Benetzung in die Hohlfasern und, wahlweise, in den dialysierflüssigkeitsseitigen Raum des Dialysators geleitet.

Die Behandlung des Dialysators mit der Sterilisationsflüssigkeit erfolgt unter Bedingungen, unter denen wenigstens die Blutseite des Dialysators sterilisiert wird. Im Verlauf oder am Ende des Sterilisationsvorgangs wird die Membran des Dialysators, beispielsweise die Hohlfasermembran, mit der Sterilisationsflüssigkeit benetzt, beispielsweise indem die Sterilisationsflüssigkeit in die Membran eingepresst wird, so dass die Poren der Membran mit der wässrigen Sterilisationsflüssigkeit gefüllt sind. Die Bedingungen zur Sterilisation hängen von der jeweils eingesetzten Sterilisationsflüssigkeit ab, insbesondere von Art und Konzentration des in der wässrigen Lösung gelösten Sterilisationsmittels, beispielsweise des Peroxids. Üblicherweise beträgt die Einwirkzeit der erfindungsgemäß als Sterilisationsflüssigkeit verwendeten wässrigen Lösungen auf den Dialysator und die Membran wenigstens 1 min und liegt beispielsweise im Bereich von 1 min bis 10 h, vorteilhaft im Bereich von 1 min bis 7,5 h. Die Behandlung erfolgt gewöhnlich bei einer Temperatur im Bereich von 10 bis 60°C, vorteilhaft von 15 bis 40°C, beispielsweise von 18 bis 25°C (Umgebungstemperatur). Allgemein kann die Einwirkzeit umso kürzer sein, je höher die Konzentration des Sterilisationsmittels in der Lösung, je niedriger der pH der Lösung und je höher die Temperatur ist. So liegt die Einwirkzeit der Sterilisationsflüssigkeit beispielsweise bei einer 7,5%igen wässrigen Wasserstoffperoxidlösung bei einer Temperatur zwischen 15 und 60°C vorteilhaft zwischen 1 min und 6 h und bei einer 0,2%igen Peroxyessigsäurelösung vorteilhaft zwischen 10 und 30 min.

Nach der Sterilisation und der Benetzung der Membran wird die Sterilisationsflüssigkeit aus dem Blutraum entfernt, beispielsweise durch Ausblasen, und der Dialysator wird in bekannter Weise einer Funktionsprüfung unterzogen, bei dem die Integrität, d. h. die Dichtigkeit, der benetzten Membran, beispielsweise der Hohlfasermembran, getestet wird. Hierzu wird die mit der Sterilisationsflüssigkeit benetzte Membran üblicherweise einseitig, insbesondere blutseitig, durch ein Prüfgas wie Luft mit Druck beaufschlagt, und nach einer Stabilisierungsphase kann die Integrität der Membran durch Messen des Druckabfalls nach einem bestimmten Zeitraum gegenüber dem Anfangsdruck, beispielsweise nach 10 s, in bekannter Weise getestet werden. Eine Integritätsprüfung kann beispielsweise auch mittels Bubble-Point-Test oder Diffusionstest oder mittels bekannter Varianten davon erfolgen. Verfahren und Vorrichtungen zur Integritätsprüfung von Membranen sind beispielsweise in US 5 594 161 A1 und WO 2012/020048 A1 beschrieben.

Wenn der Dialysator die Integritätsprüfung bestanden hat, kann sich wahlweise ein Trocknungsschritt anschließen, bei dem die wässrige Sterilisationsflüssigkeit, beispielsweise durch Erhitzen und/oder Ausblasen, entfernt wird. Die Trocknung kann in an sich bekannter Weise erfolgen. Ein Trocknen unter Erhitzen erfolgt in der Regel bei Temperaturen von bis zu 110°C, üblicherweise bis zu 105°C. Bei diesen Trocknungstemperaturen zerfallen in der wässrigen Lösung gelöste Sterilisationsmittel wie Peroxide oder Ozon zu Wasser, Sauerstoff und/oder flüchtigen organischen Verbindungen, die ebenso wie gelöste Halogene durch die Dialysemembran, beispielsweise die Hohlfasern, entweichen können. Gemäß einer Ausführungsform der Erfindung kann die Trocknung eine Mikrowellentrocknung umfassen, gegebenenfalls mit anschließender Trocknung durch vorab über Sterilfilter gereinigte Heißluft. Zur Aufrechterhaltung steriler Bedingungen können Ein- und Auslassöffnungen beispielsweise mit Kappen verschlossen werden. Verfahren und Vorrichtungen zum Trocknen von Dialysatoren, wie Mikrowellentrocknung, sind dem Fachmann bekannt und beispielsweise in DE 10 2006 051 656 A1 beschrieben.

Das erfindungsgemäße Verfahren erlaubt es, eine kombinierte Sterilisation und Integritätsprüfung von Dialysatoren unter Verwendung nur eines flüssigen Mediums durchzuführen. Nach dem Trocknen verbleiben keine toxischen Rückstände im Dialysator, so dass vor Anwendung des Dialysators kein weiterer Spülvorgang mehr erforderlich ist. Sterilisation und Integritätsprüfung können außerdem beim Hersteller selbst durchgeführt werden, so dass sowohl die Kosten als auch der logistische Aufwand reduziert werden können. Mit dem erfindungsgemäßen Verfahren lässt sich eine gute Hämokompatibilität der Dialysatoroberflächen erreichen, wobei keine vollständige Sterilisation des Dialysators erforderlich ist, sondern auch eine Sterilisation lediglich des dem Blut ausgesetzten Raums des Dialysators möglich ist.

## Patentansprüche

1. Verfahren zur Sterilisation und Integritätsprüfung von Dialysatoren mit einer Dialysemembran, einer Blutseite und einer Dialysierflüssigkeitsseite, wobei eine Sterilisationsflüssigkeit ausgewählt ist aus:
(i) wässrigen Lösungen, die Peroxid oder Ozon enthalten, wobei das Peroxid ausgewählt ist aus Peroxiden, die sich zu Wasser, Sauerstoff oder flüchtigen organischen Verbindungen zersetzen; oder
(ii) Chlor, Brom oder Iod enthaltenden wässrigen Lösungen,
wobei
das Verfahren die folgenden Schritte umfasst:
a) Behandeln des Dialysators mit der Sterilisationsflüssigkeit zur Sterilisation der Dialysierflüssigkeits- und Blutseite des Dialysators und zum beidseitigen Benetzen der Dialysemembran im Verlauf des Sterilisationsvorgangs des Dialysators mit der Sterilisationsflüssigkeit; und
b) Durchführen einer Integritätsprüfung der mit der Sterilisationsflüssigkeit beiderseits benetzten Dialysemembran
und **gekennzeichnet dadurch, dass** die Sterilisation und Integritätsprüfung eine kombinierte Sterilisation und Integritätsprüfung ist, die unter Verwendung nur eines flüssigen Mediums durchgeführt wird und wobei das nur eine flüssige Medium die Sterilisationsflüssigkeit ist.

2. Verfahren nach Anspruch 1, wobei die Sterilisationsflüssigkeit eine wässrige Lösung von ein oder mehreren Peroxiden der Formel R¹-O-O-R² ist, worin R¹ und R² unabhängig voneinander ausgewählt sind aus H, CH₃, C₂H₅ und CH₃C(O).

3. Verfahren nach Anspruch 1 oder 2, wobei die Sterilisationsflüssigkeit eine wässrige Lösung von Wasserstoffperoxid (H₂O₂) ist.

4. Verfahren nach Anspruch 3, wobei die wässrige Lösung von Wasserstoffperoxid eine 3 - 35%ige Wasserstoffperoxidlösung ist.

5. Verfahren nach Anspruch 3 oder 4, wobei die Wasserstoffperoxidlösung weiterhin Peroxyessigsäure enthält.

6. Verfahren nach einem der Ansprüche 1 bis 5, wobei die Sterilisation bei einer Temperatur im Bereich von 15 bis 60°C durchgeführt wird.

7. Verfahren nach einem der Ansprüche 1 bis 6, wobei der Dialysator zur Integritätsprüfung der Dialysemembran in Schritt (b) einseitig durch ein Prüfgas mit Druck beaufschlagt wird und die Integrität der Membran durch Messung des Druckabfalls geprüft wird.

8. Verfahren nach einem der Ansprüche 1 bis 6, wobei die Integritätsprüfung der Dialysemembran in Schritt (b) mittels Bubble-Point-Test oder Diffusionstest erfolgt.

9. Verfahren nach einem der Ansprüche 1 bis 8, wobei das Verfahren nach der Integritätsprüfung in Schritt (b) weiterhin den Schritt umfasst: c) Trocknen des Dialysators.

10. Verfahren nach einem der Ansprüche 1 bis 9, wobei der Dialysator ein Hohlfaserdialysator ist.

11. Verwendung einer Sterilisationsflüssigkeit, die ausgewählt ist aus:
(i) wässrigen Lösungen, die Peroxid oder Ozon enthalten, wobei das Peroxid ausgewählt ist aus Peroxiden, die sich zu Wasser, Sauerstoff oder flüchtigen organischen Verbindungen zersetzen; oder
(ii) Chlor, Brom oder Iod enthaltenden wässrigen Lösungen,
zur kombinierten Sterilisation und Integritätsprüfung von Dialysatoren nach einem
Verfahren gemäß einem der Ansprüche 1 bis 10.

## Claims

1. A method of sterilizing and testing the integrity of dialyzers having a dialysis membrane, a blood side and a dialysis fluid side, wherein a sterilization fluid is selected from:
(i) aqueous solutions containing peroxide and/or ozone, wherein the peroxide is selected from peroxides which disintegrate into water, oxygen and/or volatile organic compounds; or
(ii) aqueous solutions containing chlorine, bromine or iodine,
the method comprising the following steps:
a) treating the dialyzer with a sterilization fluid for sterilizing the dialysis fluid side and the blood-side of the dialyzer and for wetting the dialysis membrane on both sides in the course of sterilizing the dialyzer with sterilization fluid; and
b) carrying out an integrity testing of the dialysis membrane wetted on both sides with the sterilization fluid
and **characterized in that**
the sterilization and integrity testing is a combined sterilization and integrity testing performed by using only one fluid medium, the only one fluid medium being the sterilization fluid.

2. The method according to claim 1, wherein the sterilization fluid is an aqueous solution of one or more peroxides of the formula R¹-O-O-R², with R¹ and R² being selected independently of each other from H, CH₃, C₂H₅ and CH₃C(O).

3. The method according to claim 1 or 2, wherein the sterilization fluid is an aqueous solution of hydrogen peroxide (H₂O₂).

4. The method according to claim 3, wherein the aqueous solution of hydrogen peroxide is a 3 to 35% hydrogen peroxide solution.

5. The method according to claim 3 or 4, wherein the hydrogen peroxide solution further contains peroxy acetic acid.

6. The method according to any of claims 1 to 5, wherein the sterilization is carried out at a temperature ranging from 15 to 60°C.

7. The method according to any of claims 1 to 6, wherein for testing the integrity of the dialysis membrane, in step (b) the dialyzer is pressurized with a test gas on one side and the integrity of the membrane is tested by measuring the pressure drop.

8. The method according to one of claims 1 to 6, wherein the integrity of the dialysis membrane is tested in step (b) by means of the bubble-point test or diffusion test.

9. The method according to any of claims 1 to 8, wherein after the integrity testing in step (b) the method further comprises the step of:
c) drying the dialyzer.

10. The method according to any of claims 1 to 9, wherein the dialyzer is a hollow fiber dialyzer.

11. Use of a sterilization fluid which is selected from:
(i) aqueous solutions containing peroxide or ozone, wherein the peroxide is selected from peroxides which disintegrate into water, oxygen or volatile organic compounds; or
(ii) aqueous solutions containing chlorine, bromine or iodine,
for combined sterilization and integrity testing of dialyzers in accordance with a method according to any of claims 1 to 10.

## Revendications

1. Procédé de stérilisation et de contrôle d'intégrité de dialyseurs ayant une membrane de dialyse, un côté sang et un côté liquide de dialyse, un liquide de stérilisation étant choisi parmi :
(i) des solutions aqueuses qui contiennent du peroxyde ou de l'ozone, le peroxyde étant choisi parmi les peroxydes, qui se décomposent en eau, oxygène ou composés organiques volatiles ; ou
(ii) des solutions aqueuses contenant du chlore, du brome ou de l'iode,
ledit procédé comprenant les étapes suivantes consistant à :
a) traiter le dialyseur avec le liquide de stérilisation pour la stérilisation du côté liquide de dialyse et du côté sang du dialyseur et pour l'humidification des deux côtés de la membrane de dialyse au cours de l'opération de stérilisation du dialyseur avec le liquide de stérilisation ; et
b) réaliser un contrôle d'intégrité de la membrane de dialyse humidifiée des deux côtés avec le liquide de stérilisation
et **caractérisé en ce que**
la stérilisation et le contrôle d'intégrité est une stérilisation et un contrôle d'intégrité combinés, qui sont réalisés en utilisant seulement un milieu liquide et où le seul milieu liquide est le liquide de stérilisation.

2. Procédé selon la revendication 1, dans lequel le liquide de stérilisation est une solution aqueuse d'un ou de plusieurs peroxydes de formule R¹-O-O-R², où R¹ et R² sont choisis indépendamment les uns des autres parmi H, CH₃, C₂H₅ et CH₃C(O).

3. Procédé selon la revendication 1 ou 2, dans lequel le liquide de stérilisation est une solution aqueuse de peroxyde d'hydrogène (H₂O₂).

4. Procédé selon la revendication 3, dans lequel la solution aqueuse de peroxyde d'hydrogène est une solution de peroxyde d'hydrogène de 3 à 35 %.

5. Procédé selon la revendication 3 ou 4, dans lequel la solution aqueuse de peroxyde d'hydrogène contient en outre de l'acide peroxyacétique.

6. Procédé selon l'une des revendications 1 à 5, dans lequel la stérilisation est réalisée à une température dans la plage de 15 à 60°C.

7. Procédé selon l'une des revendications 1 à 6, dans lequel le dialyseur est soumis à un contrôle d'intégrité de la membrane de dialyse à l'étape (b) d'un seul côté par un gaz de contrôle avec de la pression et l'intégrité de la membrane est contrôlée par mesure de la chute de pression.

8. Procédé selon l'une des revendications 1 à 6, dans lequel le contrôle d'intégrité de la membrane de dialyse à l'étape (b) s'effectue au moyen du test du point de bulle ou du test de diffusion.

9. Procédé selon l'une des revendications 1 à 8, le procédé comprenant en outre après le contrôle d'intégrité à l'étape (b), l'étape consistant à :
c) sécher le dialyseur.

10. Procédé selon l'une des revendications 1 à 9, dans lequel le dialyseur est un dialyseur à fibres creuses.

11. Utilisation d'un liquide de stérilisation qui est choisi parmi :
(i) des solutions aqueuses qui contiennent du peroxyde ou de l'ozone, le peroxyde étant choisi parmi les peroxydes, qui se décomposent en eau, oxygène ou composés organiques volatiles ; ou
(ii) des solutions aqueuses contenant du chlore, du brome ou de l'iode,
pour la stérilisation et le contrôle d'intégrité combinés d'après un procédé selon l'une des revendications 1 à 10.
